(19) Europäisches Patentamt
European Patent Office
Office européen des brevets

(11) Publication number: **0 055 046**
**B1**

(12) **EUROPEAN PATENT SPECIFICATION**

(45) Date of publication of patent specification: **07.11.84**

(21) Application number: **81305736.1**

(22) Date of filing: **04.12.81**

(51) Int. Cl.³: **C 01 B 33/28,** C 01 B 33/20, C 01 B 35/12, C 01 G 17/00, C 01 G 28/00, C 01 G 31/00, C 01 G 37/14, C 01 G 39/00, C 01 G 45/12, C 01 G 49/00, B 01 J 29/28 // B01J29/36

# (54) Zeolites.

(30) Priority: **19.12.80 GB 8040782**

(43) Date of publication of application: **30.06.82 Bulletin 82/26**

(45) Publication of the grant of the patent: **07.11.84 Bulletin 84/45**

(84) Designated Contracting States: **DE FR GB NL**

(56) References cited:
**FR-A-2 130 429**
**US-A-3 308 069**
**US-A-4 061 717**
**US-E- 28 341**

(73) Proprietor: **IMPERIAL CHEMICAL INDUSTRIES PLC**
**Imperial Chemical House Millbank**
**London SW1P 3JF (GB)**

(72) Inventor: **Whittam, Thomas Vincent**
**30 Wilton Drive**
**Darlington Cleveland (GB)**

(74) Representative: **Martin, David Lincoln et al**
**Imperial Chemical Industries PLC Legal Department: Patents PO Box 6 Bessemer Road**
**Welwyn Garden City Herts, AL7 1HD (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 055 046 B1

## Description

The present invention relates to a zeolite, hereinafter referred to as zeolite Nu—2, to a method of making it and to processes using it as a catalyst.

According to the present invention we provide a synthetic zeolite material, designated zeolite Nu—2, having a molar composition expressed by the formula:

$$0.5 \text{ to } 1.8\ R_2O : Y_2O_3 : \text{at least } 10\ XO_2 : 0 \text{ to } 100\ H_2O$$

wherein R is a monovalent cation or l/n of a cation of valency n, X is silicon and/or germanium, Y is one or more of aluminium, iron, chromium, vanadium, molybdenum, arsenic, manganese, gallium, or boron, and $H_2O$ is water of hydration additional to water notionally present when R is H, and having an X-ray pattern substantially as set out in Tables 1 and 2 (as determined by standard technique using copper K$\alpha$ radiation). Table 1 shows X-ray data for zeolite Nu—2 as prepared, and Table 2 shows X-ray data for zeolite Nu—2 in the calcined Na—H form.

The X-ray data given in Tables 1 and 2 are very typical of all zeolite Nu—2 products, showing significant and rather unique features hitherto not reported for zeolites. The X-ray diffraction data show seven peaks which are always very broad, and twelve peaks which are always very sharp. The peak at d spacing 3.95 $\pm$ 0.05A is always broad based and has a sharp peak. The area under the broad diffraction peaks divided by the area under the sharp peaks is a constant irrespective of synthesis route, composition of product or temperature of preparation, and from the moment crystals of Nu—2 can be detected even at low percentages in amorphous gel reactants, these differences are constant.

Electron micrographs suggest that zeolite Nu—2 is a single crystal phase, with crystal size typically between 0.2 to 2 microns. There is no evidence for thin sheets, which could account for broad diffraction lines. A possible explanation is that the structure of Nu—2 builds up with regular faulting over a very limited range before return to the basic framework. Such faults could give rise to substantial line broadening of diffraction peaks relevant to faulting.

On calcination there are no significant changes in d spacings, but there are some changes in line intensity. The calcined sample (Table 2) was further heated at 950°C for 3 hours in air (previously saturated with water vapour at 25°C). No significant changes occurred in X-ray diffraction data. Thus even with 70% of its cation sites filled with sodium ($0.70Na_2O$, $Al_2O_3$, $49SiO_2$) zeolite Nu—2 is thermally stable to at least 950°C. To our knowledge no prior art alkali or alkaline earth zeolite shows such remarkable stability.

TABLE 1

Zeolite Nu–2 as made (Example 1)

| dA | 11.33 | 9.04 | 7.56 | 6.61 | 6.03 | 5.37 | 4.51 | 4.14 | 3.96 | 3.51 | 3.46 |
|---|---|---|---|---|---|---|---|---|---|---|---|
| 100I/Io | 23 vb | 3 | 4 vb | 3 | 3 vb | 5 vb | 2 | 23 | 100 vb* | 12 | 3 |

| dA | 3.38 | 3.31 | 3.10 | 3.02 | 2.93 | 2.91 | 2.68 | 2.59 |
|---|---|---|---|---|---|---|---|---|
| 100/I Io | 2 | 21 | 7 vb | 21 | 8 | 5 vb | 6 | 3 |

Vb = very broad diffraction peak

Vb* = very broad base but terminating in a very sharp peak

TABLE 2

Calcined sodium hydrogen Nu–2 (Example 2)

| dA | 11.33 | 9.04 | 7.56 | 6.61 | 6.03 | 5.37 | 4.51 | 4.14 | 3.96 | 3.51 | 3.46 | 3.38 | 3.31 | 3.10 | 3.02 | 2.93 | 2.91 | 2.68 | 2.59 |
|---|---|---|---|---|---|---|---|---|---|---|---|---|---|---|---|---|---|---|---|
| 100I/Io | 22 vb | 17.5 | 4 vb | 3 | 3 vb | 0 | 2 | 12 | 100 vb* | 12 | 3 | 2 | 15 | 7 vb | 12 | 3 | 5 vb | 6 | 2 |

Within the above definition of chemical composition, the number of moles of $XO_2$ is typically in the range 10 to 100 and zeolite Nu—2 appears to be most readily formed in a state of high purity when the number of moles of $XO_2$ is in the range 25 to 50.

This definition includes both freshly prepared zeolite Nu—2 ("freshly prepared" means the product of synthesis and washing, with optional drying, as hereinafter described) and also forms of it resulting from dehydration, and/or calcination, and/or ion exchange. In freshly prepared zeolite Nu—2, R may include an alkali metal cation; especially sodium, and/or ammonium, and always includes nitrogen-containing organic cations such as alkylated quaternary ammonium, phosphonium or sulphonium, or cationic degradation products thereof, or precursors thereof, or mixtures of such compounds. These cations are hereinafter referred to as Q.

The freshly prepared zeolite Nu—2 may also contain basic compounds well in excess of the 1.8 moles set out in the aforesaid definition of the composition of zeolite Nu—2, typically in the range 0.1 to 10 moles per mole of $Y_2O_3$. Since Nu—2 is a zeolite, the excess base must be physically trapped within the crystal lattice, because it is too large to escape. It can only be removed by thermal or oxidative degradation. This physically trapped basic material does not constitute part of the composition for the purposes of the definition. Thus a zeolite Nu—2 as made typically has the following molar composition:

$$0 \text{ to } 1.5\ M_2O : 0.1 \text{ to } 10\ QZ : Y_2O_3 : 10 \text{ to } 100\ XO_2 : 0 \text{ to } 100\ H_2O$$

wherein M is an alkali metal or ammonium, $Q^+$ is a quaternary ammonium or phosphonium or ternary sulphonium ion and Z is $OH^-$ or any acid group.

The $H_2O$ content of freshly prepared zeolite Nu—2 depends on the conditions in which it has been dried after synthesis.

In calcined forms of zeolite Nu—2, R may be alkali metal but includes less or no base-containing organic compounds, since these are burnt out in the presence of air, leaving hydrogen as the other balancing cation.

Among the ion-exchanged forms of zeolite Nu—2 the ammonium ($NH_4^+$) is of importance since it can be readily converted to the hydrogen form by calcination. The hydrogen form can also be prepared directly by exchange with an acid. The hydrogen-form and forms containing metals introduced by ion exchange are described further below.

We believe that zeolite Nu—2 is a member of the zeolite-beta family of zeolites. Zeolite Nu—2 can only be prepared with a much narrower range of product composition than zeolite-beta, despite the fact that it can be prepared from a wider range of reaction mixture compositions than zeolite-beta. For example zeolite Nu—2 can only be prepared in pure form with products having $SiO_2/Al_2O_3$ ratios from 20 to about 50, whereas zeolite-beta can be prepared with $SiO_2/Al_2O_3$ ratios from 5 to 100. Further, zeolite-beta can have a maximum of only 4 moles water per mole $Al_2O_3$, whereas zeolite Nu—2 can have up to 100 moles of water per mole $Al_2O_3$. Zeolite Nu—2 can be prepared from reaction mixtures

far richer in $Na_2O$ than zeolite-beta, ie the $Na_2O$/TEAOH ratio can be from 0.15 to 2.0 as compared with 0 to 0.1 for zeolite-beta. Further, the $SiO_2/Al_2O_3$ ratio for reaction mixtures giving zeolite Nu—2 can be from 10 to at least 2167 (see Example 1).

In Table 3, X-ray data for as-made zeolite Nu—2 and sodium hydrogen Nu—2 are compared with zeolite-beta data from Table 2 of US patent Reissue 28,341. Clearly the two zeolites are closely related. While many peaks are close or coincide, there are significant diferences especially in line intensities. One very important feature of Nu—2 is that as prepared, there are always 7 very broad X-ray diffraction peaks and 11 very sharp peaks. The major peak at a d spacing 3.96 has a very broad base, but a very sharp peak. Zeolite Nu—2 is to our knowledge, the only zeolite showing such unusual characteristics.

There is no indication in the aforesaid US patent or general literature that zeolite-beta has such characteristics. We believe that the very broad peaks of zeolite Nu—2 may result from regular faulting of the framework by a relatively small linking unit.

Zeolite Nu—2 has molecular sieve properties analogous to those of known zeolites. Thus zeolites Nu—2 may be characterised by its adsorption capacity for molecules of various sizes and by significant changes in these properties by removing residual alkali cation. Typical results are shown in Table 4. Slight sorption of cyclohexane and rapid sorption of p-xylene suggest an entry port size of about 0.6 nm in diameter. The results given in Table 4 also show that m-xylene is sorbed more slowly than p-xylene, thereby showing that zeolite Nu—2 will be useful for separting xylene isomers. It will also be seen from Table 4 that zeolite Nu—2 has significant hydrophobic voidage, the voids available for water being only 12.8 $cm^3$ per 100 g as compared with 18.5 $cm^3$ per 100 g for n-hexane and 18.7 $cm^3$ per 100 g for p-xylene. This means that zeolite Nu—2 is a useful sorbent for removing hydrocarbons from wet-gas or from aqueous effluents.

TABLE 3

X-ray comparison

| Zeolite | | Nu–2 | | Zeolite-beta | | | | | | | |
|---|---|---|---|---|---|---|---|---|---|---|---|
| As made Example 1 | | Calcined Example 2 | | As made dried 100°C (212°F) | | As made dried 110°C (230°F) | | As made dried 110°C (230°F) | | Calcined 538°C (1000°F) | |
| dA | RI | dA | RI | dA | RI | dA | RI | dA | RI | dA | RI |
| – | – | – | – | 14.3 | MW | – | – | – | – | – | – |
| 11.33 | 23 vb | 11.33 | 22vb | 11.5 | S | 11.5 | S | 11.5 | S | 11.55 | S |
| 9.04 | 3 | 9.05 | 17.5 | – | – | – | – | – | – | – | – |
| 7.56 | 4vb | 7.56 | 4vb | – | – | 7.56 | W | 7.59 | W | 7.5 | MW |
| – | – | – | – | 6.86 | MW | 6.97 | VW | 7.15 | W | – | – |
| 6.61 | 3 | 6.61 | 3 | 6.615 | VW | – | – | 6.54 | W | 6.59 | MW |
| – | – | – | – | – | – | 6.10 | VW | – | – | 6.14 | VW |
| 6.03 | 3vb | 6.03 | 3vb | – | – | – | – | 6.04 | W | 6.04 | VW |
| – | – | – | – | 5.64 | M | – | – | – | – | – | – |
| – | – | – | – | 5.51 | W | – | – | – | – | – | – |
| 5.37 | 5vb | – | – | 5.36 | VW | – | – | – | – | – | – |
| – | – | – | – | 5.00 | MW | – | – | – | – | – | – |
| – | – | – | – | – | – | 4.91 | VW | 4.955 | VW | – | – |
| – | – | – | – | – | – | – | – | – | – | 4.81 | VW |
| – | – | – | – | 4.75 | W | – | – | 4.73 | W | – | – |
| 4.51 | 2 | – | – | – | – | – | – | – | – | – | – |

TABLE 3 (Continued)

X-ray comparison

| Zeolite | | Nu–2 | | Zeolite-beta | | | | | | | |
|---|---|---|---|---|---|---|---|---|---|---|---|
| As made Example 1 | | Calcined Example 2 | | As made dried 100°C (212°F) | | As made dried 110°C (230°F) | | As made dried 110°C (230°F) | | Calcined 538°C (1000°F) | |
| dA | RI | dA | RI | dA | RI | dA | RI | dA | RI | dA | RI |
| – | – | – | – | 4.29 | M | – | – | 4.25 | M | – | – |
| 4.14 | 23 | 4.14 | 12 | 4.17 | M | 4.16 | M | 4.16 | M | 4.15 | M |
| 3.96 | 100vb* | 3.96 | 100vb* | 3.98 | VS | 3.97 | VS | 3.97 | VS | 3.97 | VS |
| – | – | – | – | – | – | – | – | 3.86 | MW | – | – |
| 3.51 | 12 | 3.51 | 12 | 3.54 | VW | 3.53 | W | 3.53 | M | 3.51 | MW |
| 3.46 | 3 | 3.46 | 3 | 3.42 | W | – | – | 3.46 | MW | – | – |
| 3.38 | 2 | 3.38 | 2 | – | – | – | – | – | – | – | – |
| 3.31 | 21 | 3.31 | 15 | 3.33 | MW | – | – | 3.79 | M | 3.31 | MW |
| – | – | – | – | 3.21 | VW | – | – | – | – | – | – |
| 3.10 | 7vb | 3.10 | 7vb | 3.12 | W | 3.10 | W | 3.095 | MW | 3.095 | MW |
| 3.02 | 21 | 3.02 | 12 | 3.03 | M | 3.03 | MW | 3.01 | M | 3.015 | M |
| 2.93 | 8 | 2.93 | 3 | – | – | – | – | – | – | – | – |
| 2.91 | 5vb | 2.91 | 5vb | 2.90 | M | – | – | – | – | – | – |
| – | – | – | – | 2.74 | M | – | – | – | – | – | – |
| 2.68 | 6 | 2.68 | 6 | 2.69 | W | 2.69 | VW | 2.68 | MW | 2.69 | W |
| 2.59 | 2 | 2.59 | 2 | 2.58 | W | – | – | – | – | 2.57 | VW |

Vb = very broad vb* = very broad but terminating in a very sharp peak.

TABLE 4

Sorption at 25°C, p/po = 0.5

| Adsorbate | Kinetic* diameter ρ nm | Time Min. | Wt. sorbed g/100 g | Voidage available $cm^3$/100g |
|---|---|---|---|---|
| Water | 0.27 | 10 | 7.6 | 7.6 |
| | | 60 | 12.8 | 12.8 |
| | | 1440 | 12.8 | 12.8 |
| n-hexane | 0.43 | 10 | 11.8 | 17.9 |
| | | 60 | 12.1 | 18.3 |
| | | 1440 | 12.2 | 18.5 |
| p-xylene | 0.585 | 10 | 12.7 | 14.6 |
| | | 60 | 16.3 | 18.7 |
| | | 120 | 16.3 | 18.7 |
| | | 1440 | 16.2 | 18.6 |
| m-xylene | 0.595 | 1440 | 3.5 | 4.0 |
| cyclohexane | 0.60 | 10 | 0.2 | 0.26 |
| | | 60 | 0.5 | 0.6 |
| | | 120 | 0.5 | 0.6 |
| | | 1440 | 1.0 | 1.3 |

*Lennard Jones kinetic diameter see D. W. Breck.

"Zeolite Molecular Sieves", Wiley Interscience, 1974, p.636.

Unlike zeolite-beta, a similar sodium loading i.e. about 60% of cation sites filled by Na, zeolites Nu—2 sorbs only 1% w/w cyclohexane, whereas zeolite-beta sorbs 19.4% w/w. (Table 1, US patent 3,308,068). Thus, with a 60% loading of sodium ions, zeolite Nu—2 has port sizes reduced to very close to 0.6 nm whereas zeolite-beta has ports nearer to 0.65 nm.

The sodium hydrogen Nu—2 of Example 10 of composition 0.6 $Na_2O$, $Al_2O_3$, 19.6 $SiO_2$ had substantially the same sorption characteristics as shown in Table 4, i.e. the cyclohexane capacity was only 1.2% w/w after 1440 mins. On the other hand when the product was acid exchanged twice as in Example 10, then there were very significant changes in sorption behaviour. The product composition was 0.008 $Na_2O$, $Al_2O_3$, 21 $SiO_2$ and its X-ray diffraction data were substantially as for sodium hydrogen Nu—2 of Table 2. In Table 5 typical sorption dates for hydrogen Nu—2 is given:

TABLE 5

Sorption on HNu–2

| Adsorbate | Kinetic diameter σ nm | Time Min. | Wt. sorbed g/100 g | Voidage available $cm^3$/100 g |
|---|---|---|---|---|
| Water | 0.27 | 1440 | 13.6 | 13.6 |
| p-xylene | 0.585 | 1440 | 17.0 | 19.5 |
| m-xylene | 0.595 | 1440 | 15.9 | 18.3 |
| cyclohexane | 0.60 | 1440 | 14.6 | 19.0 |
| symtrimethyl benzene | 0.78 | 1440 | 0.8 | 0.9 |

Thus, when hydrogen zeolite Nu—2 has less than 1% of its cation sites filled with sodium, cyclohexane has ready access. These results show that by altering the level of sodium in zeolite Nu—2 the ports size can be varied between about 0.60 nm and 0.65 nm.

Nu—2 has a very large voids volume, as indicated by the large quantities of organic basic compounds plus water found in all as made samples as well as large sorptive capacity. Typically, the weight loss resulting from calcining as made products is in the range 25 to 35% w/w. The substantial voids volume of sodium hydrogen Nu—2 available to molecules up to about kinetic diameter 6A is only exceeded by zeolites X, Y and beta. However, sodium zeolite Nu—2 is markedly hydrophobic, as indicated by slow initial water sorption and by the ratio

$$\frac{\text{volume n-hexane sorbed}}{\text{volume water sorbed}} = 1.45.$$

The ratio for hydrophilic zeolites such as X and Y is about 0.7.

A wide range of hydrocarbon conversion catalysts can be prepared from zeolite Nu—2 by ion exchange or impregnation with cations, or oxides, selected from the following, Cu, Ag, Mg, Ca, Sr, Zn, Cd, B, Al, Sn, Pb, V, P, Sb, Cr, Mo, W, Mn, Re, Fe, Co, Ni, noble metals. Usually the Nu—2 catalyst will be in acid form, thus stoichiometry is maintained by $H^+$ or $H_3{}^+O$ as an additional balancing cation, or as sole cation. Such catalysts are useful in the following processes; catalytic cracking, hydrodesulphurisation, hydrodenitrification, catalytic dewaxing, alkylation of alkanes or aromatics, dealkylation, disproportionation, isomerization of alkenes and alkyl benzenes, hydrocracking, catalytic cracking, dehydration reactions, oxidation and polymerization.

Zeolite Nu—2 is particularly effective in the synthesis of substituted ethers such as methyl tertiary butyl ether by reaction of olefins with alcohols as shown in our European patent application No. 55045.

As a result of hydrophobicity Nu—2 is a useful absorbent for removing hydrocarbons from aqueous streams, either liquid or gaseous.

This invention provides also a method of making zeolite Nu—2, which comprises reacting an aqueous mixture containing at least one oxide $XO_2$, at least one oxide $Y_2O_3$, and at least one alkylated or partially alkylated quaternary ammonium or phosphonium or ternary sulphonium compound i.e. $(R_1R_2R_3R_4N)^+$ or $(R_1R_2R_3R_4P)^+$ or $(R_1R_2R_3S)^+$ hereinafter referred to as $Q^+$, characterised in that the aqueous mixture has the molar composition:

| | | |
|---|---|---|
| $XO_2/Y_2O_3$ | $\geqslant$ | 10, preferably 10 to 3000. |
| $Ak^+/Q^+$ | = | 0.15 to 2.0 |
| $H_2O/QZ$ | = | 30 to 75 |
| $OH^-/XO_2$ | = | 0.1 to 2.0 |
| $H_2O/Ak^+$ | $\geqslant$ | 15 |
| $QZ/XO_2$ | = | 0.02 to 0.4 |

wherein X can be silicon and/or germanium, and Y can be aluminium, iron, chromium, vanadium, molybdenum, arsenic, manganese, gallium, boron; $Ak^+$ is an alkali metal ion, or mixtures of such ions, which can include ammonium, and refers to free alkali; $OH^-$ includes free alkali and free quaternary ammonium hdyroxide; Z is $OH^-$ or any acid radical. When Z is an acid radical an equivalent excess of free $Ak^+$ must be added as hydroxide in order to maintain the alkalinity of the reaction mixture. $Q^+$ is a quaternary ion of N, P or S. In the aforesaid definition, $R_1$, $R_2$, $R_3$ and $R_4$ can be from two to four ethyl groups, the remainder can be H, $CH_3$ or $C_3H_7$. Alternatively precursors of the quaternary compound can be used in the form of the corresponding alkylamine plus an alkanol, alkyl halide or sulphate, e.g. triethylamine plus ethanol, or an ethyl halide or sulphate, in which case the precursor is preferably preheated in a solvent e.g. methyl ethyl ketone, prior to the addition of other reactants.

The preferred alkali metal (M) is sodium. The preferred oxide $XO_2$ is silica ($SiO_2$) and the preferred oxide $Y_2O_3$ is alumina ($Al_2O_3$). The preferred quaternary compound is tetra ethyl ammonium hydroxide or its salts.

The silica source can be any of those commonly considered for use in synthesising zeolites, for example powdered solid silica silicic acid, colloidal silica or dissolved silica, especially those made by precipitation from an alkali metal silicate solution, such as the type known at "KS 300" made by AKZO, and similar products, aerosil silicas, fume silicas and silica gels suitable in grades for use in reinforcing pigments for rubber or silicone rubber. Colloidal silicas of various particle sizes may be used, for example 10—15 or 40—50 microns, as sold under the Registered Trade Marks "LUDOX", "NALCOAG" and "SYTON". The usable dissolved silicas include commercially available waterglass silicates containing 0.5 to 6.0, especially 2.0 to 4.0 mols of $SiO_2$ per mol of alkali metal oxide, "active" alkali metal silicates as defined in UK Patent 1193254, and silicates made by dissolving silica in alkali metal hydroxide or quaternary ammonium hydroxide or a mixture thereof.

The alumina source is most conveniently sodium aluminate, but can be or include an aluminium or aluminium salt for example the chloride, nitrate or sulphate or alumina itself, which should preferably be in a hydrated or hydratable form such as colloidal alumina, pseudoboehmite, boehmite, gamma alumina or the alpha or beta trihydrate.

The reaction mixture is reacted under autogenous pressure or with added nitrogen presure if desired, at temperatures between 85 and 250°C until crystals of zeolite Nu—2 form, which can be from 1 hour to many months depending on the reactant composition and the operating temperature. Agitation is optional, but preferable since it reduces the reaction time.

At the end of the reaction, the solid phase is collected on a filter and washed and is then ready for further steps such as drying, dehydration and ion-exchange.

If the product of the reaction contains alkali metal ions, these have to be at least partly removed in order to prepare the hydrogen form of Nu—2 and this can be done by ion exchange with an acid, especially a strong mineral acid such as hydrochloric acid or by way of the ammonium compound, made by ion exchange with a solution of an ammonium salt such as ammonium chloride. Such ion exchange can be carried out by slurrying once or several times with the solution. The zeolite is usually calcined after ion exchange and may be calcined before or between stages.

In general, the cation(s) of zeolite Nu—2 can be replaced by any cation(s) of metals, and particularly those in Groups IA, IB, IIA, IIB, III (including rare earths VIII) (including noble metals) and by lead, tin and bismuth. (The Periodic Table is as in "Abridgements of Specifications" published by the UK Patent Office). Exchange is carried out using any water soluble salts containing the appropriate cation.

In order to prepare a catalyst, zeolite Nu—2 can be incorporated in an inorganic matrix, with other materials which can be either inert or catalytically active. The matrix may be present simply as a binding agent to hold the small zeolite particles (0.005 to 10 microns) together, or it may be added as a diluent to control the amount of conversion in a process which may otherwise proceed at too high a rate, leading to catalyst fouling as a result of excessive coke formation. Typical inorganic diluents include catalyst support materials such as alumina, silica and kaolinic clays, bentonites, montmorillonites, sepiolite, attapulgite, Fullers earth, synthetic porous materials such as $SiO_2$—$Al_2O_3$, $SiO_2$—$ZrO_2$, $SiO_2$—$ThO_2$, $SiO_2$—BeO, $SiO_2$—$TiO_2$ or any combination of these oxides. An effective way of mixing zeolite Nu—2 with such diluents is to mix appropriate aqueous slurries in a mixing nozzle and then to

spray-dry the slurry. Other ways of mixing can be used. The invention is illustrated by the following examples. Examples 1, 3, 5, 7 and 8 are very unusual in that a crystalline zeolite was crystallised from a clear solution, and not in the conventional manner, namely from a gel/slurry.

Example 1

The synthesis mixture had the following molar composition:

$$342\ Q_2O,\ 556\ Na_2O,\ Al_2O_3,\ 2167\ SiO_2,\ 35{,}000\ H_2O$$

481 g of 30% colloidal silica (Syton X30 of composition $Na_2O$, 0.04 $Al_2O_3$, 85.6 $SiO_2$, 689 $H_2O$) were stirred into a prepared solution of 45.8 g sodium hydroxide in 435 g tetra ethyl ammonium hydroxide (25% aqueous solution). The clear solution was reacted in a stainless steel vessel with stirring for 28 days at 95°C. The product was washed and dried overnight at 120°C and had the following compositions.

$$4.6\ Q_2O,\ 0.7\ Na_2O,\ Al_2O_3,\ 48\ SiO_2,\ 15.5\ H_2O$$

X-ray analysis showed the product was crystalline zeolite Nu—2 of this invention (see Table 1).

Example 2

The product of Example 1 was calcined in air (saturated with water vapour at 25°C) for 48 hours at 450°C. The weight loss was 35% and the product was a sodium hydrogen Nu—2 of composition.

$$0.7\ Na_2O,\ Al_2O_3,\ 49\ SiO_2$$

This calcined product had X-ray diffraction data as shown in Table 2, again typical of zeolite Nu—2 but with some changes in line intensities, when compared with the as-made zeolite.

Example 3

The experiment of Example 1 was repeated except that the operating temperature was 150°C and the reaction time to crystallize Nu—2 was 48 hours. The product analysis was

$$0.9\ Na_2O,\ 3.5\ Q_2O,\ Al_2O_3,\ 39\ SiO_2,\ 12\ H_2O$$

Example 4

The synthesis mixture had the following molar composition

$$12.8\ Na_2O,\ 7.5\ Q_2O,\ Al_2O_3,\ 50\ SiO_2,\ 500\ H_2O$$

11 g of silica (Akzo KS300 — 7.18 $Na_2O$, $Al_2O_3$, 69.5 $SiO_2$, 226 $H_2O$) were suspended in 294 g tetra ethyl ammonium hydroxide (25% aqueous solution). A solution containing 29.7 g sodium hydroxide, and 7.2 g sodium aluminate (1.25 $Na_2O$, $Al_2O_3$ 3 $H_2O$) was added with stirring. The mixture was reacted in a stainless steel vessel at 95°C for 32 days. The product was crystalline Nu—2 of composition.

$$0.9\ Na_2O,\ 2.5\ Q_2O,\ Al_2O_3,\ 28\ SiO_2,\ 9\ H_2O$$

Example 5

The synthesis mixture had the following molar composition.

$$3.6\ Na_2O,\ 20\ Q_2O,\ Al_2O_3,\ 154\ SiO_2,\ 1534\ H_2O$$

257 g KS300 silica were dissolved in 588 g tetra ethyl ammonium hydroxide (25% aqueous solution). Then a solution containing 4.6 g sodium aluminate and 2.0 g sodium hydroxide in 216 g water was added with stirring. The resulting clear solution was reacted at 95°C until zeolite Nu—2 crystallised out i.e. for 28 days.

Example 6

The synthesis mixture had the following molar composition.

$$40.6\ Na_2O,\ 30\ QI,\ Al_2O_3,\ 100\ SiO_3,\ 1570\ H_2O$$

184.5 g of triethyl sulphonium iodide were dissolved in 250 g water, and then stirred into 485 g of sodium silicate solution ($Na_2O$, 0.01 $Al_2O_3$, 3.77 $SiO_2$, 24 $H_2O$). Finally a solution of 25.6 g sodium hydroxide and 5.8 g sodium aluminate in 63 g water was added with stirring. The resulting slurry was reacted for 3 days at 150°C in a stirring stainless steel autoclave. The product was crystallined zeolite Nu—2 of composition.

$$Na_2O,\ Q_2O,\ Al_2O_3,\ 25.6\ SiO_2,\ 12\ H_2O$$

Example 7

The synthesis mixture had the following molar composition.

$$1.0\ Na_2O,\ 24.6\ K_2O,\ 14.8\ Q_2O,\ Al_2O_3,\ 100\ SiO_2,\ 1570\ H_sO$$

260 g KS300 silica were dissolved in 682 g tetraethyl ammonium hydroxide solution (25% aqueous solution). Next 5.3 g Kaiser SA alumina powder ($Al_2O_3$ 3 $H_2O$) were dissolved in 106.8 g potassium hydroxide in 590 g water. The clear solution was reacted for 24 days at 95°C after which zeolite Nu—2 crystallised from solution.

Example 8

The synthesis mixture had the following molar composition.

$$100\ Na_2O,\ 57.4\ Q_2O,\ Al_2O_3,\ 377\ SiO_2,\ 5966\ H_2O$$

A clear solution containing 361 g sodium silicate, 211 g tetra ethyl ammonium hydroxide (40% aqueous solution) and 189 g water was reacted for 48 hours at 150°C in a stirred stainless steel autoclave. Towards the end of the run, i.e. from 40 hours, zeolite Nu—2 crystallised out. The product after washing with litres of water and drying overnight at 120°C had the following molar composition.

$$0.7\ Na_2O,\ 2.8\ Q_2O,\ Al_2O_3,\ 50\ SiO_2,\ 25\ H_2O$$

Example 9

The synthesis mixture had the following molar composition.

$$1.35\ Na_2O,\ 3.14\ Q_2O,\ Al_2O_3,\ 29\ SiO_2,\ 311\ H_2O$$

8.7 g solid sodium hydroxide were dissolved in 250 g tetra ethyl ammonium hydroxide (40% aqueous solution followed by 16.4 g Kaiser SA alumina powder. Next 649 g colloidal silica was added with stirring. The resulting gel/slurry was crystallised to zeolite Nu—2 in a stirred autoclave after 6 days at 150°C. The washed dried product had the molar composition

$$0.6\ Na_2O,\ 2.2\ Q_2O,\ Al_2O_3,\ 20\ SiO_2,\ 6\ H_2O$$

One sample of this material was calcined for 3 hours in air at 950°C. The product had the X-ray diffraction pattern of Table 3 and composition 0.6 $Na_2O$, $Al_2O_3$, 19.6 $SiO_2$. Which further illustrates that sodium Nu—2 is exceptionally thermally stable.

Example 10

A second sample of the zeolite Nu—2 of Example 9 was calcined for 48 hours at 550°C in air, (saturated with water at 25°C). The product was exchanged with 5 ml Normal hydrochloric acid per gram of zeolite by slurrying for 1 hour at 60°C. The product was washed and dried overnight at 120°C and after calcining for 3 hours at 450°C had the following molar composition

$$0.2\ Na_2O,\ Al_2O_3,\ 20.5\ SiO_2.$$

This sample ws subjected to a repeat ion exchange with Normal hydrochloric acid and the final product had the composition.

$$0.008\ Na_2O,\ Al_2O_3,\ 21\ SiO_2$$

i.e. was effectively a hydrogen zeolite containing less than 0.03% w/w Na, and its X-ray diffraction data were substantially as shown in Table 2 (i.e. similar to sodium hydrogen Nu—2 of example 2).

Example 11

This example illustrates the use of HNu—2 as a catalyst in toluene disproportionation. Approximately 2 g of the product of Example 9 was calcined in air for 16 hours at 450°C. The resulting material was compressed, crushed and sieved. 0.440 g of 250—500 $\mu$ particle size HNu—2 so prepared was loaded into a microreactor and tested for its ability to disproportionate toluene. The catalyst was flushed with nitrogen for two hours, over which time the temperature of the reactor was raised to 530°C. Toluene was then contacted with the catalyst at 530°C by feeding at a rate equivalent to a WHSV of 10.4. The results of this reaction are given in Table 6 below.

TABLE 6

| Time on stream hours | Toluene Conversion wt, % | wt. % p-xylene in xylenes fraction |
|---|---|---|
| 1.2 | 10.7 | 39.8 |
| 2.5 | 4.0 | 49.2 |

It will be evident from the data in Table 6 that the amount of p-xylene in the xylenes fraction is considerably higher than its equilibrium concentration of 24 percent.

## Claims

1. A synthetic zeolite material having a molar composition expressed by the formula:

$$0.5 \text{ to } 1.8\ R_2O : Y_2O_3 : \text{at least } 10\ XO_2 : 0 \text{ to } 100\ H_2O$$

wherein R is a monovalent cation or l/n of a cation of valency n, X is silicon and/or germanium, Y is one or more of aluminium, iron, chromium, vanadium, molybdenum, arsenic, manganese, gallium or boron, and $H_2O$ is water of hydration additional to water notionally present when R is H and having, as made, an X-ray diffraction pattern substantially as shown in Table 1.

2. A synthetic zeolite material according to claim 1 having a molar composition expressed by the formula:

$$0.5 \text{ to } 1.8\ R_2O : Y_2O_3 : 10 \text{ to } 100\ XO_2 : 0 \text{ to } 100\ H_2O.$$

3. A synthetic zeolite material according to claim 2 having a molar composition expressed by the formula:

$$0.5 \text{ to } 1.8\ R_2O : Y_2O_3 : 25 \text{ to } 50\ XO_2 : 0 \text{ to } 100\ H_2O.$$

4. A synthetic zeolite material according to claim 1 having, as freshly made, a molar composition expressed by the formula:

$$0 \text{ to } 1.5\ M_2O : 0.1 \text{ to } 10\ QZ : Y_2O_3 : 10 \text{ to } 100\ XO_2 : 0 \text{ to } 100\ H_2O$$

wherein M is an alkali metal or ammonium, $Q^+$ is a quaternary ammonium or phosphonium or ternary sulphonium ion and Z is $OH^-$ or any acid group.

5. A synthetic zeolite material according to any one of claims 1 to 3 wherein R is or includes hydrogen.

6. A method of making a synthetic zeolite as defined in claim 1 which comprises reacting an aqueous mixture comprising at least one oxide $XO_2$, at least one oxide $Y_2O_3$ and at least one alkylated or partially alkylated quaternary ammonium or phosphonium or ternary sulphonium compound or a precursor thereof in the form of the corresponding alkylamine plus an alkanol, alkyl halide or sulphate, characterised in that the aqueous mixture has the molar composition:

| | |
|---|---|
| $XO_2/Y_2O_3$ | at least 10 |
| $Ak^+/Q^+$ | 0.15 to 2.0 |
| $H_2O/QZ$ | 30 to 75 |
| $OH^-/XO_2$ | 0.1 to 2.0 |
| $H_2O/Ak^+$ | at least 15 |
| $QZ/XO_2$ | 0.02 to 0.4 |

wherein X, Y, Z and Q have the meanings given in claims 1 and 4 and $Ak^+$ is an alkali metal or ammonium ion.

7. A method according to claim 6 wherein $XO_2/Y_2O_3$ is in the range 10 to 3000.

8. A method according to claim 6 or 7 wherein the quaternary ammonium compound is tetraethylammonium hydroxide.

9. A catalyst comprising a synthetic zeolite material as claimed in any one of claims 1 to 5.

10. A catalytic process employing the catalyst claimed in claim 9.

**Revendications**

1. Matière zéolitique synthétique ayant une composition molaire exprimée par la formule:

$$0,5 \text{ à } 1,8\ R_2O : Y_2O_3 : \text{au moins } 10\ XO_2 : 0 \text{ à } 100\ H_2O$$

où R est un cation monovalent ou la n-ième partie d'un cation de valence n, X est le silicium et/ou le germanium, Y représente un ou plusieurs des éléments aluminium, fer, chrome, vanadium, molybdène, arsenic, manganèse, gallium ou bore, et $H_2O$ désigne l'eau d'hydratation en plus de l'eau théoriquement présente lorsque R représente H et ayant, telle que préparée, un diagramme de diffraction de rayons X correspondant principalement au tableau I.

2. Matière zéolitique synthétique suivant la revendication 1, ayant une composition molaire exprimée par la formule:

$$0,5 \text{ à } 1,8\ R_2O : Y_2O_3 : 10 \text{ à } 100\ XO_2 : 0 \text{ à } 100\ H_s(H_2O$$

3. Matière zéolitique synthétique suivant la revendication 2, ayant une composition molaire exprimée par la formule:

$$0,5 \text{ à } 1,8\ R_2O : Y_2O_3 : 25 \text{ à } 50\ XO_2 : 0 \text{ à } 100\ H_2O.$$

4. Matière zéolitique synthétique suivant la revendication 1, ayant, telle que fraîchement préparée, une composition molaire exprimée par la formule:

$$0 \text{ à } 1,5\ M_2O : 0,1 \text{ à } 10\ QZ : Y_2O_3 : 10 \text{ à } 100\ XO_2 : 0 \text{ à } 100\ H_2O$$

où M est un métal alcalin ou l'ion ammonium, $Q^+$ est un ion ammonium ou phosphonium quaternaire ou un ion sulfonium tertaire et Z représente $OH^-$ ou tout groupe acide.

5. Matière zéolitique synthétique suivant l'une quelconque des revendications 1 à 3, dans laquelle R est ou renferme de l'hydrogène

6. Procédé de préparation d'une zéolite synthétique telle que décrite dans la revendication 1, qui consiste à faire réagir un mélange aqueux comprenant au moins un oxyde $XO_2$, au moins un oxyde $Y_2O_3$ et au moins un composé d'ammonium ou de phosphonium quaternaire ou de sulfonium ternaire alkylé ou partiellement alkylé, ou un précurseur de ce composé sous la forme de l'alkylamine correspondante plus un alcanol, un halogénure d'alkyle ou un sulfate d'alkyle, caractérisé en ce que le mélange aqueux a la composition molaire:

| | |
|---|---|
| $XO_2/Y_2O_3$ | au moins 10 |
| $Ak^+/Q^+$ | 0,15 à 2,0 |
| $H_2O/QZ$ | 30 à 75 |
| $OH^-/XO_2$ | 0,1 à 2,0 |
| $H_2O/Ak^+$ | au moins 15 |
| $QZ/XO_2$ | 0,02 à 0,4 |

dans laquelle X, Y, Z et Q ont les définitions données dans les revendications 1 et 4 et $Ak^+$ est un ion de métal alcalin ou d'ammonium.

7. Procédé suivant la revendication 6, dans lequel $XO_2/Y_2O_3$ se situe dans l'intervalle de 10 à 3000.

8. Procédé suivant la revendication 6 ou 7 dans lequel le composé d'ammonium quaternaire est l'hydroxyde de tétraéthylammonium.

9. Catalyseur comprenant une matière zéolitique synthétique suivant l'une quelconque des revendications 1 à 5.

10. Procédé catalytique utilisant le catalyseur suivant la revendication 9.

**Patentansprüche**

1. Synthetisches Zeolithmaterial mit einer molaren Zusammensetzung, die durch die folgende Formel ausgedrückt wird:

$$0{,}5 \text{ bis } 1{,}8\ R_sO : Y_2O_3 : \text{mindestens } 10\ XO_2 : 0 \text{ bis } 100\ H_2O$$

worin R ein einwertiges Kation oder 1/n eines Kations mit der Wertigkeit n ist, X Silicium und/oder Germanium ist, Y ein oder mehr als ein Vertreter von Aluminium, Eisen, Chrom, Vanadium, Molybdän, Arsen, Mangan, Gallium oder Bor ist und $H_2O$ Hydratwasser ist, das zu Wasser hinzukommt, das begrifflich vorhanden ist, wenn R H ist, wobei das synthetische Zeolithmaterial in der Form, in der es hergestellt ist, ein Röntgenbeugungsbild liefert, wie es im wesentlichen in Tabelle 1 gezeigt wird.

2. Synthetisches Zeolithmaterial nach Anspruch 1 mit einer molaren Zusammensetzung, die durch die Formel:

$$0{,}5 \text{ bis } 1{,}8\ R_2O : Y_2O_3 : 10 \text{ bis } 100\ XO_2 : 0 \text{ bis } 100\ H_2O$$

ausgedrückt wird.

3. Synthetisches Zeolithmaterial nach Anspruch 2 mit einer molaren Zusammensetzung, die durch die Formel:

$$0{,}5 \text{ bis } 1{,}8\ R_2O : Y_2O_3 : 25 \text{ bis } 50\ XO_2 : 0 \text{ bis } 100\ H_2O$$

ausgedrückt wird.

4. Synthetisches Zeolithmaterial nach Anspruch 1, das, wenn es frisch hergestellt ist, eine molare Zusammensetzung hat, die durch die Formel:

$$0 \text{ bis } 1{,}5\ M_2O : 0{,}1 \text{ bis } 10\ QZ : Y_2O_3 : 10 \text{ bis } 100\ XO_2 : 0 \text{ bis } 100\ H_2O$$

ausgedrückt wird, worin M ein Alkalimetall oder Ammonium ist, $Q^+$ ein quaternäres Ammonium- oder Phosphonium- oder einternäres Sulfoniumion ist und Z $OH^-$ oder irgendeine saur Gruppe ist.

5. Synthetisches Zeolithmaterial nach einem der Ansprüche 1 bis 3, worin R Wasserstoff ist oder Wasserstoff enthält.

6. Verfahren zur herstellung eines synthetischen Zeoliths, wie er in Anspruch 1 definiert ist, bei dem eine wäßrige Mischung, die mindestens ein Oxid $XO_2$, mindestens ein Oxid $Y_2O_3$ und mindestens eine alkylierte oder partiell alkylierte quaternäre Ammonium-oder Phosphonium- oder ternäre Sulfoniumverbindung oder eine Vorstufe davon in Form des entsprechenden Alkylamins zuzüglich eines Alkanols, Alkylhalogenids oder -sulfats enthält, zur Reaktion gebracht wird, dadurch gekennzeichnet, daß die wäßrige Mischung die folgende molare Zusammensetzung hat:

| | |
|---|---|
| $XO_2/Y_2O_3$ | mindestens 10 |
| $Ak^+/Q^+$ | 0,15 bis 2,0 |
| $H_2O/QZ$ | 30 bis 75 |
| $OH^-/XO_2$ | 0,1 bis 2,0 |
| $H_2O/Ak^+$ | mindestens 15 |
| $QZ/XO_2$ | 0,02 bis 0,4 |

worin X, Y, Z und Q die in der Ansprüchen 1 und 4 angegebenen Bedeutungen haben und $Ak^+$ ein Alkalimetall- oder Ammonium ion ist.

7. Verfahren nach Anspruch 6, bei dem das Verhältnis $XO_2/Y_2O_3$ in dem Bereich von 10 bis 3000 liegt.

8. Verfahren nach Anspruch 6 oder 7, bei dem die quaternäre Ammoniumverbindung Tetraethylammoniumhydroxid ist.

9. Katalysator, der ein synthetisches Zeolithmaterial nach einem der Ansprüche 1 bis 5 enthält.

10. Katalytisches Verfahren, bei dem der Katalysator nach Anspruch 9 eingesetzt wird.